(19) Europäisches Patentamt
European Patent Office
Office européen des brevets

(11) **EP 2 488 215 B1**

(12) **EUROPÄISCHE PATENTSCHRIFT**

(45) Veröffentlichungstag und Bekanntmachung des Hinweises auf die Patenterteilung:
**14.08.2013 Patentblatt 2013/33**

(21) Anmeldenummer: **10763697.9**

(22) Anmeldetag: **08.10.2010**

(51) Int Cl.:
*A61L 2/10* *(2006.01)*   *A61L 2/00* *(2006.01)*

(86) Internationale Anmeldenummer:
**PCT/EP2010/065109**

(87) Internationale Veröffentlichungsnummer:
**WO 2011/045243 (21.04.2011 Gazette 2011/16)**

(54) **VERFAHREN ZUR INAKTIVIERUNG VON UNERWÜNSCHTEN KONTAMINATIONEN IN BLUTEGELEXTRAKTEN**

METHOD FOR DEACTIVATING UNDESIRED CONTAMINATIONS IN LEECH EXTRACTS

PROCÉDÉ POUR L'INACTIVATION DE CONTAMINATIONS INDÉSIRABLES DANS DES EXTRAITS DE SANGSUE

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **13.10.2009 EP 09172861**

(43) Veröffentlichungstag der Anmeldung:
**22.08.2012 Patentblatt 2012/34**

(73) Patentinhaber: **Bayer Intellectual Property GmbH 40789 Monheim (DE)**

(72) Erfinder:
• **SCHMIDT, Sebastian**
**42781 Haan (DE)**
• **PETERS, Jörg**
**42781 Haan (DE)**
• **MICHELS, Jürgen**
**51061 Köln (DE)**

(74) Vertreter: **BIP Patents**
**c/o Bayer Intellectual Property GmbH**
**Creative Campus Monheim**
**Alfred-Nobel-Straße 10**
**40789 Monheim (DE)**

(56) Entgegenhaltungen:
**EP-A1- 1 676 818    WO-A1-02/38502**
**WO-A2-02/38191    BE-A- 415 573**

**Beschreibung**

Die Erfindung betrifft das Gebiet der Inaktivierung von Viren und/oder Bakterien mittels elektromagnetischer Strahlung. Gegenstand der vorliegenden Erfindung ist ein Verfahren zur Inaktivierung von Viren und/oder Bakterien in Blutegelextrakten.

Blutegel werden seit der Antike zur medizinischen Therapie eingesetzt. Ihre große Blutaufnahmekapazität wurde bereits im alten Griechenland und besonders im Mittelalter zur medizinischen Entnahme von Blut aus dem Körper eingesetzt (Aderlass).

Zu Beginn des 19. Jahrhunderts kamen Blutegelextrakte auf dem Markt, die gerinnungshemmende Wirkungen aufwiesen. Im Jahr 1955 wurde erstmalig ein Polypeptid mit dem Namen Hirudin aus Blutegeln extrahiert. Hirudin bindet sich an die Fibrinogenbindestelle von Thrombin und hemmt über einen Ausläufer das aktive Zentrum, wodurch dessen Wirkung blockiert wird.

Eine historische Übersicht zu Blegelextrakten und Hirudin gibt die folgende Veröffentlichung: Nowak, G. & Schrör, K. (2007): Hirudin - the long and stony way from an anticoagulant peptide in the saliva of medicinal leech to a recombinant drug and bevond. A historical piece; in: Thromb. Haemost. Bd. 98, S. 116-119.

Zur Gewinnung von therapeutisch wirksamen Blutegel-Substanzen werden gefrorene Blutegel (z.B. *Hirudo medicinalis, Hirudo verbana und dazu verwandte Arten*) oder Bestandteile davon mechanisch zerkleinert und homogenisiert. In einem mehrstufigen Extraktions- und Reinigungsverfahren kann ein Wirkstoff gewonnen werden, der beispielsweise in einer Salbe zur Behandlung von Venenschwäche und akuten hämorrhidalen Beschwerden eingesetzt werden kann.

Da es sich bei Blutegelextrakten um ein Produkt aus einer natürlichen Rohstoffquelle handelt, ist die Sicherheit vor ungewollten Kontaminationen wie Bakterien oder Viren von hoher Bedeutung. Bei der Implementierung eines Virussicherheitskonzeptes ist ausdrücklich der Einsatz komplementärer, also sich im Wirknechanismus ergänzender Technologien vorgeschrieben (siehe z. B. Guideline Q5A der *International Conference on Harmonisation of Technical Requirements for Registration of Pharmaceuticals for Human Use (ICH)*). Damit soll sichergestellt werden, dass ein breites Spektrum an Viren erfasst wird.

Etablierte Verfahren zur Inaktivierung von Bakterien und großen umhüllten Viren sind saure Behandlung, Behandlung mit organischen Lösemitteln, Behandlung mit Detergenzien und Pasteurisierung, die einzeln oder vorzugsweise in Kombination eingesetzt werden.

Für kleine nicht umhüllte Viren wie z.B. Parvoviren sind diese Methoden allerdings nicht wirksam.

Ein Verfahren zur Abreicherung kleiner nicht umhüllter Viren stellt die Nanofiltration dar. Dabei erfolgt die Abtrennung durch Größenausschluss: eine Membran hält Viren, deren Größe innerhalb der angegebenen Abscheiderate liegen, sicher zurück.

Bei der Nanofiltration besteht jedoch das Problem, das kleine nicht-umhüllte Viren eine ähnliche Größe wie die gewünschte therapeutische Substanz haben können. Dadurch ist es also nicht möglich, die Erreger sicher herauszufiltern, nicht aber das Protein. Die Folge ist ein erhöhter, inakzeptabler Produktverlust, der zu einem nicht mehr wirtschaftlichen Verfahren führt.

Bei der Nanofiltration von Blutegelextrakten zeigte sich in eigenen Untersuchungen, dass die erreichbare Flussrate pro Membranfläche extrem gering ist. Gleichzeitig kam es schon nach sehr kurzer Prozessierungszeit zu einer Verblockung der Filterflächen. Diese Verblockungen waren nicht reversibel. Die Filter konnten durch übliche Maßnahmen wie z.B. Rückspülen nicht wieder in einen funktionsfähigen Zustand gebracht werden. Durch diese extrem geringe Leistungsfähigkeit der Nanofiltration bei gleichzeitig hohen Kosten für eine Filtereinheit scheidet ein Nanofiltrationsverfahren zur Virusabreicherung von Blutegelextrakten für eine wirtschaftliche Anwendung in einer pharmazeutischen Produktion aus.

Ein weiteres Verfahren zur Virusinaktivierung ist die Bestrahlung mit ultraviolettem Licht (UV-Bestrahlung). Die besondere Herausforderung liegt bei diesem Verfahren in der homogenen Bestrahlung des zu prozessierenden Mediums. Zielsetzung ist die zuverlässige und weitgehende Abtötung von Mikroorganismen und/oder Viren bei gleichzeitig weitgehender Erhaltung der empfindlichen Wertstoffe. Insbesondere Produkte aus natürlichen Rohstoffquellen weisen eine komplexe, vielfältige Zusammensetzung auf. In der Regel zeigen die verschiedenen Produktbestandteile eine unterschiedliche Stabilität gegenüber UV-Bestrahlung. Dies erschwert die Kompromissfindung zwischen Virusinaktivierung und Erhaltung der Produktqualität.

Ein wichtiges Kriterium zur Produktschonung ist die Verkürzung der Produktexposition in der Bestrahlungszone. Da die notwendige mittlere Behandlungsdauer durch die am schnellsten die Bestrahlungszone passierenden Teilchen festgelegt wird, besteht zur Reduzierung der Behandlungsdauer die Forderung nach einer möglichst einheitlichen Verweilzeitverteilung innerhalb des Produktstroms. Probleme bei der Verwendung von Reaktoren zur Einstrahlung von ultraviolettem Licht in fluide Medien ergeben sich durch eine mit zunehmender Entfernung von der Strahlungsquelle exponentiell abnehmende Strahlungsintensität im zu behandelnden Medium. Mikroorganismen und Viren in einem größeren Abstand von der Strahlenquelle werden aus diesem Grund langsamer bzw. überhaupt nicht mehr abgetötet.

Dieser Effekt, der mit zunehmendem Lichtabsorptionsvermögen des Mediums erheblich verstärkt wird, führt nach dem derzeitigen Stand der Technik zur Verwendung sehr großer Bestrahlungsoberflächen, wie man sie z. B. in Dünnschichtreaktoren vorfindet. Die im Einsatz befindlichen Dünnschichtreaktoren lassen sich jedoch nur schwer in den technischen Maßstab überführen, da die Konstanthaltung der Filmdicke bei der Maßstabsvergrößerung nur durch eine durchsatzproportionale Durchmesservergrößerung zu realisieren ist, was im technischen Maßstab zu nicht mehr handhabbar großen Reaktoren führt.

Einen weiteren negativen Einfluss bildet das ungünstige Verweilzeitverhalten der nach Maßgabe der zumeist nur geringen Eindringtiefe der UV-Strahlung in das Reaktionsmedium notwendigerweise sehr dünnen und damit laminar strömenden Flüssigkeitsfilme, bei denen ein Austausch quer zur Hauptströmungsrichtung entfällt. Die wandnahen Schichten verweilen wegen des linear zur Wand bis auf Null abnehmenden Geschwindigkeitsprofils wesentlich länger als die wandfemeren Schichten. Um die zur Abtötung notwendige Mindestbestrahlungsdosis auch in der schneller fließenden wandfernen Flüssigkeitsschicht realisieren zu können, muss die mittlere Verweilzeit des Films angehoben werden. Dies aber führt zu einer erhöhten Strahlenbelastung und somit zu einer größeren Schädigung der Produkte.

In der Literatur (EP 1 339 643A1, EP 1 337 280A1) wird das besonders günstige Verweilzeitverhalten in wendelförmigen Strömungskanälen beschrieben. Ein Produkt durchströmt einen helikal geformten Strömungskanal. Durch die helikale Strömungsführung kommt es im Kanal zu Sekundärströmungen, den so genannten Dean-Wirbeln, welche eine intensive und zugleich schonende Durchmischung garantieren. Durch die hohe Mischwirkung der Wirbel wird eine enge Verweilzeitverteilung und Dosisverteilung realisiert. So ist es möglich, gezielt eine wirksame Strahlendosis einzutragen, welche ausreichend ist, um Viren zu inaktivieren, ohne das Produkt stark zu belasten. Dieses so genannte Dosiskonzept ist unabhängig von der Modulgröße, so dass der Scale-up vom Labor- in den Produktionsmaßstab gegeben ist.

Die UV-Bestrahlung in einem so genannten Wendelmodul ist prinzipiell so ausgelegt, dass eine einmalige Durchströmung durch das Wendelmodul durchgeführt wird. Je nach Trübung der zu prozessierenden Flüssigkeit kann die Flussrate im Wendelmodul innerhalb bestimmter Grenzen variiert werden. Die Grenzen ergeben sich durch die notwendige Ausbildung der Sekundärströmung und dem Druckverlust im Modul. Sollte selbst die geringste mögliche Flussrate und damit die höchste Verweilzeit nicht ausreichen, um die gewünschte Vireninaktivierung zu erreichen, wäre es prinzipiell denkbar, mehrere Module in Serie zu betreiben. Hier begrenzt jedoch der Druckverlust über die Anordnung von mehreren Modulen in Serie und die Druckstabilität der Module die entsprechende Anzahl.

Im Fall von Blutegelextrakten ist die Absorption der Flüssigkeit so hoch (optische Dichte bei 254 nm ist größer als 50 cm$^{-1}$), dass die Eindringtiefe von UV-Strahlung in einen Blutegelextrakt auf den Bereich an der Oberfläche und wenige Mikrometer darunter beschränkt ist. Eine signifikante Virusinaktivierung beim Durchlaufen eines einzelnen Wendelmoduls bei gleichzeitigem Erhalt der Produktintegrität ist nicht zu gewährleisten. Dies zeigten eigene Untersuchungen. Auch die potenzielle Möglichkeit des Betriebes mehrerer hintereinander geschalteter Module schied aus praktischen Gründen aus. Für eine ausreichende Inaktivierung wären über 4 Module in Serie notwendig gewesen. Dies wäre wegen des damit einhergehenden Druckverlustes bei der limitierten Druckbeständigkeit der Anlage in der Praxis nicht realisierbar gewesen.

Weiterhin besteht insbesondere bei den komplexen Wirkstoffgemischen in Blutegelextrakten die Gefahr der Belagbildung in der Bestrahlungszone, die den Eintrag der Strahlen in das zu bestrahlende Medium dämpfen oder sogar ganz verhindern kann.

Es muss also befürchtet werden, dass eine UV-Bestrahlung bei Blutegelextrakten keine wirksame Methode zur Virusinaktivierung darstellt. Das Problem besteht auch darin, dass eine Belagbildung nur schwer erkennbar ist, wenn das zu bestrahlende Medium eine sehr hohe optische Dichte aufweist. In einem solchen Fall ist es nicht möglich, einen Fotosensor in das zu bestrahlende Medium einzubringen und die Strahlungsintensität zu messen, um Belagbildung festzustellen. Es besteht die Gefahr, dass das zu bestrahlende Medium unzureichend bestrahlt wird und somit eine ausreichende Produktsicherheit nicht gegeben ist.

Ausgehend vom Stand der Technik stellt sich damit die Aufgabe, ein Verfahren zur Inaktivierung von Viren und/oder Bakterien, insbesondere von kleinen nicht-umhüllten Viren, in Blutegelextrakten bereitzustellen. Das gesuchte Verfahren soll zu einer höheren Produktausbeute als die klassischen Verfahren wie saure Behandlung, Lösemittelbehandlung, Detergenzbehandlung, Pasteurisation und/oder Nanofiltration führen und dabei gleichzeitig einen wirtschaftlichen Betrieb und eine hohe Produktqualität gewährleisten. Ferner soll das gesuchte Verfahren eine Möglichkeit bieten, Belagbildung zu erkennen, um eine ausreichende Produktsicherheit sicherstellen zu können.

Überraschend wurde gefunden, dass eine Virus- und Bakterieninaktivierung in Blutegelextrakten effektiv und wirtschaftlich dadurch erzielt werden kann, dass der Blutegelextrakt zwischen einem Rührkessel und einer Bestrahlungsvorrichtung, in der der Blutegelextrakt einer Bestrahlung mit ultraviolettem Licht ausgesetzt wird, im Kreislauf geführt wird.

Gegenstand der vorliegenden Erfindung ist daher ein Verfahren zur Inaktivierung von Viren und/oder Bakterien in einem fluiden Blutegelextrakt. gemäß Anspruch 1.

Bei dem fluiden Blutegelextrakt handelt es sich vorzugsweise um den mittels des in Beispiel 1 genannten Verfahrens gewonnenen Extrakt.

Unter Inaktivierung wird ein Prozess verstanden, der dazu führt, dass unerwünschte Eigenschaften der Viren und/oder Bakterien vermindert oder eliminiert werden. Die Inaktivierung erfolgt durch Bestrahlung mit ultraviolettem Licht, das bekanntermaßen dazu geeignet ist, Viren und/oder Bakterien so zu verändern, dass von ihnen keine schädigende Wirkung für Menschen, Tiere, Pflanzen und/oder Umwelt mehr ausgehen.

Unter ultraviolettem Licht wird elektromagnetische Strahlung im Wellenlängenbereich von 100 nm bis 400 nm verstanden. Für die Virusinaktivierung wird bevorzugt so genannte UV-C-Strahlung im Bereich von 100 nm bis 280 nm eingesetzt, besonders bevorzugt im Bereich von 200 nm bis 280 nm.

Erfindungsgemäß wird der Blutegelextrakt zwischen einem Rührkessel und einer Bestrahlungsvorrichtung im Kreislauf geführt. Die Bestrahlüngsvorrichtung besteht aus einem oder mehreren vorzugsweise parallel geschalteten Wendelmodulen, die weiter unten näher beschrieben sind.

Überraschenderweise wird durch die Kreislauffahrweise eine ausreichende Inaktivierung von Viren und Bakterien in Blutegelextrakten erreicht, ohne dass es zu nennenswerten Schädigungen der im Blutegelextrakt enthaltenen Proteine kommt. Die durch den Einsatz eines Rührreaktors bedingte verbreiterte Verweilzeitverteilung (im Vergleich zum einmaligen Durchlaufen von einem oder mehreren hintereinander geschalteten Wendelmodulen) und die erhöhte Bestrahlungszeit im einzelnen Modul infolge der Kreislauffahrweise haben dabei überraschenderweise für die im Blutegelextrakt enthaltenen Proteine keine schadhafte Auswirkung. Zusätzlich kommt es trotz der entsprechend langen Prozesszeit im einzelnen Wendelmodul überraschenderweise nicht zu relevanten Belag- oder Aggregatbildungen.

Überraschend wurde gefunden, dass sich in dem erfindungsgemäßen Verfahren Viren und Bakterien durch Bestrahlung mit ultraviolettem Licht sogar in Blutegelextrakten mit einer optischen Dichte größer als 70 inaktivieren lassen. Vorzugsweise werden Blutegelextrakte mit einer optischen Dichte im Bereich von 10 bis 72, besonders bevorzugt im Bereich von 30 bis 65, ganz besonders bevorzugt im Bereich von 40 bis 60 eingesetzt.

Unter optischer Dichte $OD$ (auch Extinktion genannt) wird der dekadische Logarithmus des Verhältnisses aus der Intensität $I_0$ der in ein Medium einfallender Strahlung und der Intensität I der aus dem Medium austretenden Strahlung verstanden:

$$OD = \lg (I_0/I)$$

Die optische Dichte ist abhängig von der Wellenlänge der verwendeten Strahlung. Im vorliegenden Dokument wird die optische Dichte bei einer Wellenlänge von 254 nm angegeben.

Der Blutegelextrakt wird erfindungsgemäß im Kreislauf gefahren. Das Verhältnis von umgepumptem Volumenstrom zum Gesamtvolumen liegt im Bereich von 0,5 bis 80 1/h, bevorzugt im Bereich von 1 bis 60 1/h, besonders bevorzugt im Bereich von 3 bis 45 1/h.

Die Temperatur des Extrakts wird während der Durchführung des erfindungsgemäßen Verfahrens auf einer Temperatur im Bereich von 2°C bis 25°C, bevorzugt im Bereich von 4°C bis 20°C, besonders bevorzugt im Bereich von 8°C bis 15°C gehalten.

In einer bevorzugten Ausführungsform wird bevor und/oder nachdem ein Ansatz in einer Kreislauffahrweise bestrahlt worden ist, ein oder mehrere Kreisläufe mit einem transparenten Medium gefahren und die Intensität der in der Bestrahlungszone in das transparente Medium eingetragenen oder durch das transparente Medium durchgetretenen Strahlung gemessen.

Die optische Dichte eines Blutegelextrakts ist nämlich zu hoch, um während der Bestrahlung des Extrakts eine Messung der in der Bestrahlungszone in den Blutegelextrakt eintretenden oder gar durch den Blutegelextrakt durchtretenden Strahlungsintensität messen zu können. Es besteht jedoch die Gefahr, dass es im Verlauf der Bestrahlung zu einer Belagbildung an der Innenwand eines Bestrahlungsmoduls kommt. Die Folge einer Belagbildung wäre eine verminderte in den Extrakt eintretende Strahlungsintensität. Dadurch wäre eine vollständige Inaktivierung der Viren und/oder Bakterien nicht mehr sichergestellt. Daher wird vor und/oder nach der Blutegelextraktbestrahlung ein transparentes Medium durch die Anlage gefördert und die Intensität der in das Medium eingetragenen oder durch das Medium durchgetretenen Strahlung gemessen. Ist die Strahlungsintensität vor und nach der Blutegelextraktbestrahlung gleich oder annähernd gleich, kann eine Belagbildung ausgeschlossen werden und die nächste Charge an Blutegelextrakt bestrahlt werden. Ist eine signifikante Abnahme der Strahlungsintensität zu verzeichnen, so ist es denkbar, dass sich ein Belag an der Innenwand eines Bestrahlungsmoduls gebildet hat, der vor der nächsten Charge entfernt werden sollte. Alternativ ist es auch denkbar, die Strahlungsintensität zu erhöhen und/oder die Zahl der Kreisläufe zu erhöhen, um die verminderte Strahlungsintensität entsprechend zu kompensieren.

Unter einem transparenten Medium wird ein Medium verstanden, das für einen Wellenlängenbereich der verwendeten Strahlung eine optische Dichte von kleiner als 10 aufweist (vorzugsweise gemessen bei 254 nm).

Vorzugsweise wird als transparentes Medium Wasser oder eine wässrige Pufferlösung eingesetzt. Als Pufferlösung eignet sich beispielsweise ein Phosphatgepufferte Salzlösung (PBS) oder andere organische/ anorganische Puffersysteme.

Zusätzlich zu der Belagbildung muss auch eine strahlungsbedingte Aggregatbildung in der Flüssigkeit, sowohl bezüglich des Produkts, als auch bezüglich von Nebenkomponenten oder beidem vermieden werden. Trotz der Kreislauffahrweise des Blutegelextrakts konnte in der erfindungsgemäßen Ausführung überraschend keinerlei Aggregatbildung beobachtet werden.

Das erfindungsgemäße Verfahren wird in einer Vorrichtung durchgeführt, die mindestens einen Rührkessel, eine Bestrahlungsvorrichtung und ein Fördermittel für den Blutegelextrakt umfasst.

Unter einem Rührkessel wird ein Behälter verstanden, in dem der Blutegelextrakt gelagert werden kann, und der Mittel für eine Durchmischung des im Behälter befindlichen Blutegelextrakts umfasst. Üblicherweise werden als Mittel für eine Durchmischung Rührwerke wie beispielsweise ein Blattrührer eingesetzt. Der Behälter kann beispielsweise aus Glas, Edelstahl oder einem Kunststoff bestehen.

Das Fördermittel dient der Förderung des fluiden Mediums aus dem Rührkessel durch die Bestrahlungsvorrichtung zurück in den Rührkessel. Als Fördermittel eignet sich beispielsweise eine Pumpe.

Die Vorrichtung zur Durchführung des erfindungsgemäßen Verfahrens ist dadurch gekennzeichnet, dass Rührkessel, Bestrahlungsvorrichtung und Fördermittel so miteinander verbunden sind, dass der Blutegelextrakt vom Rührkessel durch die Bestrahlungsvorrichtung und wieder zurück in den Rührkessel geführt werden kann.

Die Bestrahlungsvorrichtung umfasst ein oder mehrere vorzugsweise parallel geschaltete Wendelmodule.

Unter einem Wendelmodul wird eine Vorrichtung verstanden, die mindestens eine Quelle für elektromagnetische Strahlung und einen helikal um eine Achse verlaufenden Kanal bereitstellt. Beispiele für solche Wendelmodule sind in der Offenlegung WO 2002/038502A1 in den Figuren 5, 6, 7, 8, 9 oder 10 dargestellt. Der helikal verlaufende Kanal ist vorzugsweise so angeordnet, dass er um eine Quelle für elektromagnetische Strahlung herumläuft. Es ist denkbar, weitere Quellen für elektromagnetische Strahlung um den Kanal herum anzuordnen.

Wird ein solcher helikal verlaufender Kanal mit einem fluiden Medium durchströmt, so wirkt auf das Medium eine intensive, über die gesamte Länge des Kanals herrschende gleichförmige Quervermischung senkrecht zur Hauptrichtung der Produktströmung. Durch die Quervermischung wird trotz der beim erfindungsgemäßen Verfahren herrschenden laminaren Strömungscharakteristik eine eingeengte Verweilzeitverteilung bewirkt. Durch die Quervermischung wird weiterhin gewährleistet, dass die von der Strahlenquelle entfernteren Fluidschichten, die besonders bei stark absorbierenden Medien keine oder wenig elektromagnetische Strahlung erhalten, einen intensiven Austausch mit den bestrahlten Schichten nahe der Strahlenquelle eingehen. Dies und die enge Verweilzeitverteilung führt dazu, dass alle Fluidelemente eine gleichmäßige und einheitliche Bestrahlungsdauer und - intensität erfahren, die sich durch die Strömungsgeschwindigkeit und die Intensität der Strahlungsquelle auf die jeweiligen Bedürfnisse anpassen lassen. So kann gewährleistet werden, dass eine effektive Reduktion an Mikroorganismen und/oder Viren in dem Medium erfolgt. Bei Medien, bei denen eine zu starke Bestrahlung zu Schäden führen kann, wird die Gefahr, dass es durch eine ungünstig breite Verweilzeitverteilung zu einer zu starken Strahlenbelastung und damit teilweisen Schädigung kommt, wirksam vermindert.

Es ist denkbar, dass in einem Wendelmodul mehrere Kanäle benachbart angeordnet sind und helikal um eine gemeinsame Achse verlaufen. Ein Kanal kann ein eckiges, rundes, ovales oder halbrundes Querschnittsprofil aufweisen. Weitere Querschnittsprofile sind denkbar. Bevorzugt weist der Kanal ein Querschnittsprofil auf, das auf mindestens einer Seite abgeflacht ist. Von dieser abgeflachten Seite wird bevorzugt elektromagnetische Strahlung in den Kanal eingebracht. Beispiele für solche Kanäle sind in den Figuren 5, 6, 7, 8, 9 oder 10 in WO 2002/038502A1 gezeigt. Das Querschnittsprofil des Kanals ist vorzugsweise D-förmig (d.h. halbkreisförmig oder halbelliptisch), rautenförmig oder rechteckig.

Als Quelle für elektromagnetische Strahlung eignet sich jede Quelle, die Strahlung mit einer Wellenlänge emittiert, die zur Inaktivierung von Viren und/oder Bakterien geeignet ist. Vorzugsweise wird eine Quelle für UV-C-Strahlung wie beispielsweise eine Quecksilberdampflampe eingesetzt, die ein Strahlungsmaximum bei einer Wellenlänge von 254 nm aufweist. Es ist denkbar, mehrere Quellen für elektromagnetische Strahlung einzusetzen.

In einer besonders bevorzugten Ausführungsform umfasst das Wendelmodul einen Hohlzylinder, auf den ein Wendelschlauch kraft- oder formschlüssig aufgebracht ist. In den Hohlzylinder ist eine Quelle für elektromagnetische Strahlung ohne direkte Produktberührung eingebracht. Solche Wendelmodule sind beispielhaft in den Anmeldungen WO 02/38502A1, WO 02/38191A1, WO 07/096057A1, EP 1 464 342A1 und DE 10 2009 009 108.4 beschrieben.

In WO 07/096057A2 ist beispielsweise ein Wendelmodul beschrieben, das dadurch gekennzeichnet ist, dass über ein inneres Stützrohr ein Wendelschlauch formschlüssig aufgebracht ist. Dadurch entsteht zwischen dem Stützrohr und dem Wendelschlauch ein Kanal, der helikal von einem Ende des Wendelschlauches um das Stützrohr zum anderen Ende des Wendelschlauches verläuft. Vorzugsweise ist der Wendelschlauch kraftschlüssig auf einen Hohlzylinder aufgebracht, wie es in der Anmeldung DE 10 2009 009 108.4 beschrieben ist. Querströmungen zwischen benachbarten Kanalwindungen können so wirksam vermieden werden. Diese Querströmungen würden ansonsten zu einer ungewollten Verbreiterung der Verweilzeitverteilung führen.

Die Wendelmodule sind bevorzugt so gestaltet, das mindestens die bestrahlten Bauteile als Einwegteile ausgeführt sind.

Das Volumenverhältnis zwischen dem Rührkessel und der Bestrahlungsvorrichtung mit einem oder mehreren Wendelmodulen liegt im Bereich von 1 bis 1000, bevorzugt im Bereich von 5 bis 500, besonders bevorzugt im Bereich von 10 bis 200. Dadurch kann eine Prozesszeit eingehalten werden, die gut in den betrieblichen Ablauf, d. h. z. B. in eine Schicht eingebettet werden kann.

Die Vorrichtung zur Durchführung des erfindungsgemäßen Verfahrens ist bevorzugt mit einem oder mehreren Sensoren ausgestattet, z. B. für die Bestrahlung (z.B. UV-Sensor), den Druck, den Behälterfüllstand, die Temperatur und den Volumenstrom. Weiterhin ist die Vorrichtung vorzugsweise mit Sensoren ausgestattet, die die korrekte Einbaulage der Wendelmodule überwachen und mit Leckage-Sensoren, die eine eventuelle Undichtigkeit detektieren. In einer bevorzugten Ausführungsform sind auch Sicherheitseinrichtungen vorgesehen. Dies können z.B. sein: Maßnahmen zur Verhinderung einer ungewollten Bestrahlung des Bedienpersonals (z.B. eine Einhausung mit Türüberwachung), Auffangwannen für den Fall einer Leckage, Schutzeinrichtungen bei bewegten Apparateteilen.

Die gesamte Vorrichtung wird bevorzugt durch ein Prozessleitsystem gesteuert und geregelt. Insbesondere werden die Temperatur, die Flussrate, die Bestrahlung und die Prozesszeit überwacht.

Die Bestandteile der Vorrichtung sind vorzugsweise CIP-fähig ausgeführt (*CIP=clean in place*), um eine Sterilisierung für pharmazeutische Anwendungen sicherzustellen.

Die Erfindung wird nachstehend anhand von Beispielen näher erläutert, ohne sie hierauf zu beschränken.

Es zeigen:

Fig. 1:　Schematische Darstellung eines Verfahrens zur Herstellung eines Roh-Blutegelextrakts

Fig. 2:　Schematische Darstellung eines Verfahrens zur Herstellung eines gefriergetrockneten Blutegelextrakts

Fig. 3:　Schematische Darstellung einer Ausführungsform einer Vorrichtung zur Durchführung des erfindungsgemäßen Verfahrens

Fig. 4:　Schematische Darstellung eines Bestrahlungsmoduls

Fig. 5:　Schematische Darstellung eines Bestrahlungsmodul-Kopfes

Fig. 6:　Schematische Darstellung eines durchströmten helikalen Kanals, in dem sich Dean-Wirbel bilden

Fig. 7:　Grafische Darstellung der Inaktivierung von Hirudin und Viren in einem Medium als Funktion der Bestrahlungsdosis.

Beispiel 1: *Verfahren zur Herstellung eines Blutegelextrakts*

*Erster Verfahrensabschnitt* (*Rohextrakt-Herstellung*)

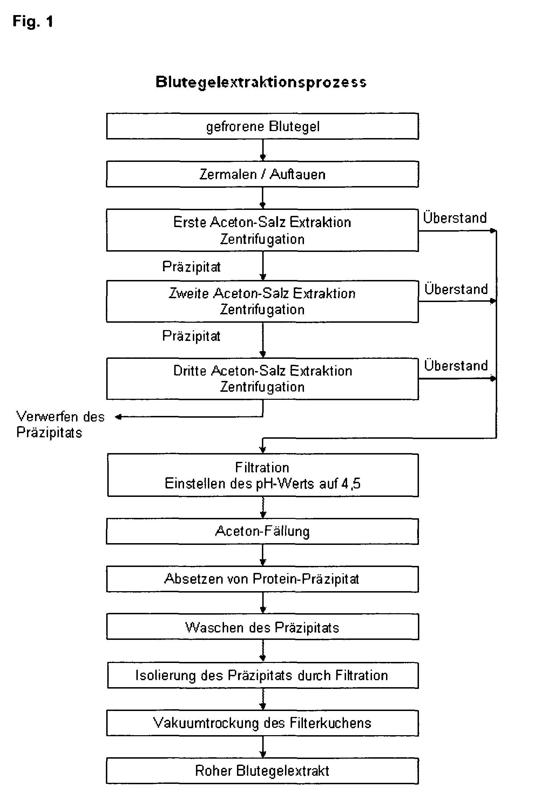

Der erste Verfahrensabschnitt zur Herstellung eines Blutegelextrakts, die Herstellung des Rohextrakts, ist in Fig. 1 schematisch dargestellt.

Tiefgefrorene Blutegel wurden angetaut und in zwei Portionen mit insgesamt 70-80 kg in einem sog. "Cutter" zerkleinert. Nach der Zerkleinerung wurde die Suspension mit erwärmtem gereinigtem Wasser verdünnt und in den Extraktionskessel transferiert, in dem das Volumen durch Zugabe von gereinigtem Wasser adjustiert wurde. Kochsalz und Aceton wurden zur ersten Extraktionsstufe hinzugefügt, während die Suspension weiter gerührt und temperiert wurde. Nach der ersten Extraktionsstufe wurde die Suspension durch Zentrifugation in eine biomassehaltige und eine flüssige Phase getrennt. Die flüssige Phase wurde zwischengelagert. Die Festphase wurde in temperiertem gereinigtem Wasser aufgenommen und erneut mit einer steigenden Kochsalz- und Acetonkonzentration extrahiert (zweite Extraktionsstufe). Danach wurde erneut zentrifugiert und schließlich wurde bei erhöhter Kochsalz- und Acetonkonzentration die dritte Extraktionsstufe durchgeführt. Die biomassehaltige Festphase wurde anschließend verworfen. Die flüssigen Phasen wurden vereinigt, filtriert und durch Zugabe von Trichloressigsäure (TCA) auf einen pH von 4-5, bevorzugt 4.5 ($\pm$0.1) eingestellt, bevor die Proteinfällung auf tiefgekühltem Aceton erfolgte. Das Proteinpräzipitat setzt sich ab und wurde anschließend von der Aceton-Oberphase betrennt. Das Präzipitat wurde dreifach mit einem Aceton-Wasser-Gemisch (80% v/v) gewaschen. Zwischen den Waschschritten setzte sich das Präzipitat ab. Anschließend wurde jeweils die Aceton-Oberphase abgetrennt. Das gewaschene Präzipitat wurde durch Filtration geerntet und mit Aceton gewaschen. Anschließend wurde das überschüssige Aceton durch Spülung mit Stickstoffgas verdrängt und der Filterkuchen entnommen. Die feuchten Filterkuchen können optional tiefgefroren zwischengelagert werden. Die Filterkuchen wurden im Vakuumtrockenschrank getrocknet, um restliches Aceton zu entfernen. Die getrockneten Filterkuchen, die den Blutegel-Rohextrakt enthalten, wurden bis zur Weiterverarbeitung tiefgefroren zwischengelagert.

*Zweiter Verfahrensabschnitt (Herstellung von Blutegelextrakt (lyophilisiert))*

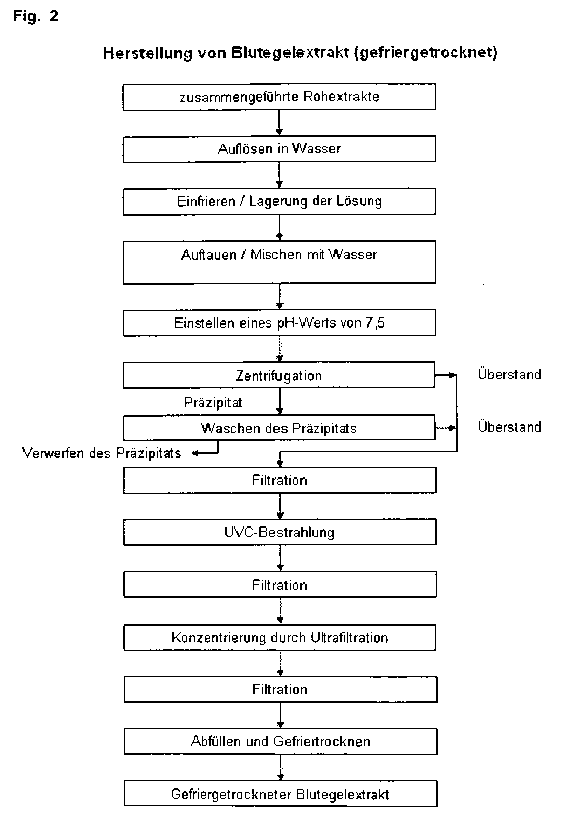

Fig. 2 zeigt schematisch den zweiten Verfahrensabschnitt zur Herstellung eines Blutegelextrakts, die Herstellung des gefriergetrockneten Blutegelextrakts.

Diverse getrocknete Filterkuchen, die den Blutegel-Rohextrakt enthalten, wurden kombiniert und in gereinigtem Wasser aufgelöst. Die entstandene Proteinlösung wurde eingefroren und in einem Tiefkühlschrank zwischengelagert. Danach wurde die Lösung wieder aufgetaut und gereinigtes Wasser zugefügt. Die verdünnte Proteinlösung wurde aufgeheizt und bei konstanter Temperatur für eine festgelegte Zeit pasteurisiert. Im Anschluss wurde die Proteinlösung auf Raumtemperatur abgekühlt und auf einen neutralen pH von 7-8, bevorzugt 7,5 ($\pm$0.1) mit verdünnter Salzsäure oder Natriumcarbonat eingestellt. Die pH-adjustierte Lösung wurde zentrifugiert und der Überstand vereinigt und gekühlt zwischengelagert. Das abgetrennte Präzipitat wurde gewaschen durch Zugabe von gereinigtem Wasser und erneuter Zentrifugation.

Die Überstände wurden nachfolgend kombiniert, homogenisiert und filtriert. Falls notwendig, kann die optische Dichte $OD$(254 nm) durch Zugabe von gereinigtem Wasser geeignet adjustiert werden. Als geeignet haben sich optische Dichten bis zu 72 erwiesen. Anschließend erfolgte eine UV-Bestrahlung bei einer Wellenlänge von 254 nm und einer geeigneten Dosis. Als geeignet haben sich Dosiswerte von 50 bis 1000 J/m$^2$ erwiesen, bevorzugt 100-600 J/m$^2$, besonders bevorzugt 250-350 J/m$^2$. Die UVbestrahlte Proteinlösung wurde nachfolgend filtriert und konzentriert (Ultrafiltration), um die Aktivität einzustellen. Die eingestellte Bulk-Lösung wurde abschließend noch einmal filtriert, in Fläschchen abgefüllt und dann gefriergetrocknet. Der gefriergetrocknete Blutegelextrakt wurde zur weiteren Verarbeitung zwischengelagert.

**Beispiel 2:** *Vorrichtung zur Inaktivierung von unerwünschten Kontaminationen in einem fluiden Medium mit einer hohen optischen Dichte von mehr als 50*

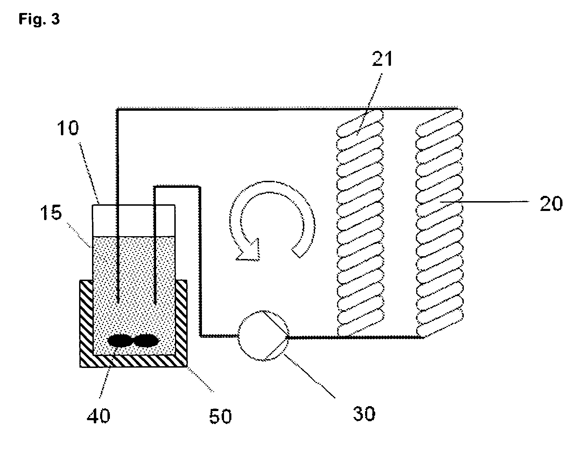

Eine Vorrichtung zur Ausführung des erfindungsgemäßen Verfahrens ist schematisch in Fig. 3 dargestellt.

Die Vorrichtung besteht im Wesentlichen aus einem Behälter 10, z.B. einem Rührkessel mit einem Rührwerk 40 und einem oder mehreren Bestrahlungsmodulen (20, 21). Das Medium 15 wird über Schlauch- oder Rohrverbindungen vom Behälter 10 durch die Bestrahlungsmodule 20, 21 und anschließend wieder zurück in den Behälter 10 geleitet. Dies geschieht vorzugsweise mit einer Pumpe 30. Die Bestrahlungsmodule können parallel oder seriell verschaltet sein oder auch kombiniert seriell und parallel.

Das Bestrahlungsmodul ist ein vorzugsweise ein Wendelmodul nach der oben genannten Definition, umfassend einen wendelförmigen Bestrahlungsraum der um eine stabförmige Strahlenquelle, die u. a. eine Wellenlänge von 254 nm abstrahlt, geführt wird.

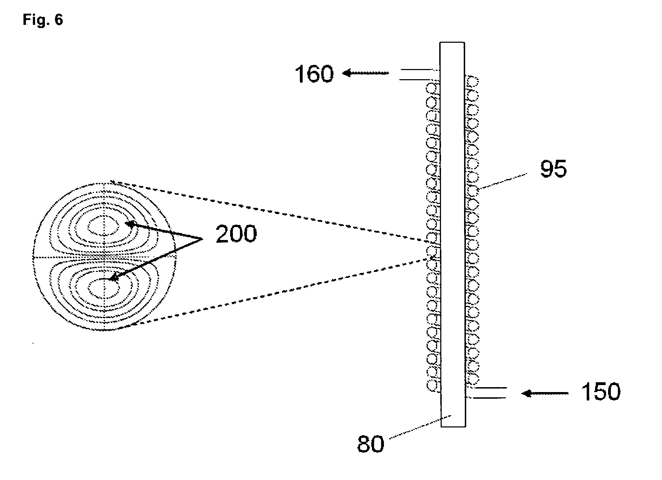

Die Strahlenquelle ist bevorzugt eine Quecksilberdampflampe. Der Bestrahlungsraum ist mindestens an der zur Strahlenquelle gerichteten Seite in einem für Strahlung mit der Wellenlänge 254 nm durchlässigen Material ausgeführt, bevorzugt aus Quarzglas. Durch die in Wendelform geführte Strömung entstehen Sekundärwirbel, sogenannte Dean-Wirbel 200 (s. Fig. 6), die eine effiziente und effektive Quervermischung der Flüssigkeit auch im laminaren Strömungsregime erzeugen. Auf diese Weise werden alle Flüssigkeitsteile im Verlauf der Durchströmung des Moduls in einer wandnahen Schicht bestrahlt. Zusätzlich wird durch diese Strömungsführung eine Verengung der Verweilzeit bewirkt.

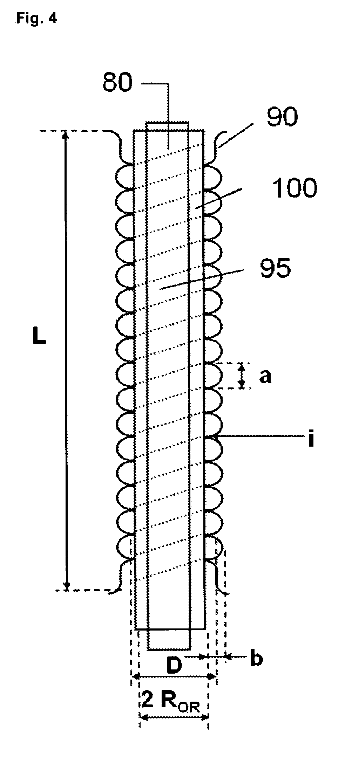

Eine bevorzugte Ausführungsform eines Wendelmoduls ist in Fig. 4 gezeigt.

In dieser bevorzugten Ausführungsform umfasst das Wendelmodul einen Teflonschlauch 90, der eine wendelförmige Kerbung und dadurch eine aufgeprägte Wendel aufweist. In diesen Teflonschlauch ist ein Quarzrohr 100 kraftschlüssig eingebracht. Durch diesen Aufbau werden die einzelnen Wendeln 95 voneinander getrennt und es entsteht ein Wendelrohrleitungssystem. Im Inneren der Quarzröhre 100 ist eine UV-Lampe 80 eingebracht. Diese Position ermöglicht es, die durch die Wendeln strömende Lösung auf ihren gesamten Weg durch den Reaktor maximal zu bestrahlen.

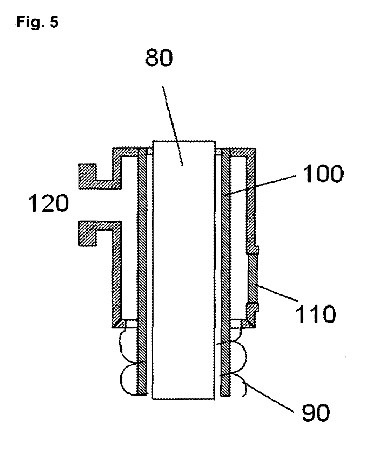

Der Flüssigkeitseintrag erfolgt bevorzugt über einen unteren Eingang und ermöglicht so einen blasenfreien Eintrag der verschiedenen Lösungen. Am unteren und oberen Ende des Bestrahlungsmoduls befindet sich jeweils ein Reaktorkopf (siehe Fig. 5), der für die Flüssigkeitszufuhr bzw. Flüssigkeitsabfuhr bestimmt ist. Durch eine eingelassene Öffnung 110 kann mittels eines UV-Sensors die Leistung der Strahlenquelle überwacht werden.

In Fig. 4 bedeuten:

D = mittlerer Durchmesser einer Wendel
b = Breite des halbelliptischen Strömungskanals
L = Länge des Teflonschlauchs
a = mittlere Höhe einer Wendel
i = Abstand zwischen zwei Wendeln
$R_{QR}$ = halber Außendurchmesser der Quarzröhre

Beispiel 3: Verfahren zur Behandlung des Blutegelextrakts aus Beispiel 1 in der Vorrichtung aus Beispiel 2

Ein Blutegelextrakt aus dem Verfahren gemäß Beispiel 1 wurde mit einer Vorrichtung gemäß Beispiel 2 mit einem Bestrahlungsmodul bestrahlt. Dazu wurde ein Volumen von 230 mL Extrakt, das mit einer kleinen Menge (>10%) Viren-Stammlösung (Minute Virus of Mice) versetzt war, vorgelegt. Die optische Dichte betrug 53,3. Das Extrakt wurde durch eine Schlauchpumpe mit 10 L/h durch ein 24 mL Bestrahlungsmodul gepumpt, dort mit UV-Licht bei 254 nm bestrahlt, und in die Vorlage zurückgeführt (Kreislauffahrweise). Nach 0, 10, 20 und 30 Minuten wurde eine Probe aus dem Vorlage entnommen. Dies entspricht einer Bestrahlungsdosis von 0, 97, 198 bzw. 303 J/m$^2$. Von jeder Probe wurde mit Standard-Assays die Virusaktivität und die Aktivität des Hirudin-Wirkstoffes im Extrakt bestimmt.

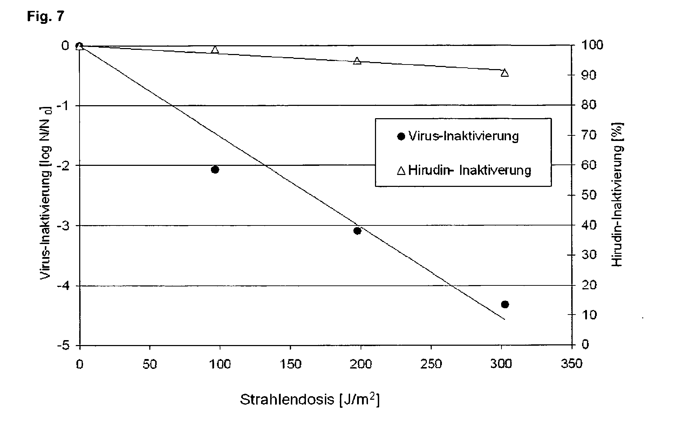

Die Ergebnisse sind in Fig. 7 grafisch dargestellt. Fig. 7 zeigt die Inaktivierung von Hirudin und der zugesetzten Viren als Funktion der Bestrahlungsdosis. Das Ergebnis verdeutlicht die klare vireninaktivierende Wirkung bei gleichzeitig geringer Schädigung des Wirkstoffes. Da die Schädigung des Wirkstoffs aber durchaus vorhanden ist, muss bei einer vorgegebenen Mindest-Vireninaktivierung eine möglichst präzise Bestrahlung gewählt werden, um unnötige Verluste zu vermeiden.

Bezugszeichen

| | |
|---|---|
| 10 | Rührkessel |
| 15 | Medium (Blutegelextrakt) |
| 20 | Wendelmodul |
| 21 | Wendelmodul |
| 30 | Fördermittel (z.B. Pumpe) |
| 40 | Rührer |
| 50 | Kühlmantel |
| 80 | Strahlungsquelle |
| 90 | Teflonschlauche |
| 95 | Wendel |
| 100 | Quarzglasrohr |
| 110 | verschließbare Öffnung zum Anbringen eines Sensors |
| 120 | Einlass/Auslass |
| 150 | Einlass |
| 160 | Auslass |

**Patentansprüche**

1. Verfahren zur Inaktivierung von Viren und/oder Bakterien in einem Blutegelextrakt, **dadurch gekennzeichnet, dass** der Blutegelextrakt zwischen einem Rührkessel und einer Bestrahlungsvorrichtung, in dem das Medium einer Bestrahlung mit ultraviolettem Licht ausgesetzt ist, im Kreis geführt wird.

2. Verfahren nach Anspruch 1, **dadurch gekennzeichnet, dass** die Bestrahlung mit ultraviolettem Licht im Bereich von 100 nm bis 280 nm, bevorzugt im Bereich von 200 nm bis 280 nm erfolgt.

3. Verfahren nach einem der Ansprüche 1 oder 2, **dadurch gekennzeichnet, dass** der Blutegelextrakt bei einer Wellenlänge von 254 nm eine optische Dichte im Bereich von bis zu 72, bevorzugt im Bereich von 30 bis 65, besonders bevorzugt im Bereich von 40 bis 60 aufweist.

4. Verfahren nach einem der Ansprüche 1 bis 3, **dadurch gekennzeichnet, dass** das Verhältnis von umgepumptem Volumenstrom zum Gesamtvolumen im Bereich von 0,5 bis 80 1/h, bevorzugt im Bereich von 1 bis 60 1/h, besonders bevorzugt im Bereich von 3 bis 45 1/h liegt.

5. Verfahren nach einem der Ansprüche 1 bis 4, **dadurch gekennzeichnet, dass** die Temperatur des Blutegelextrakts im Bereich von 2°C bis 25°C, bevorzugt im Bereich von 4°C bis 20°C, besonders bevorzugt im Bereich von 8°C bis 15°C gehalten wird

6. Verfahren nach einem der Ansprüche 1 bis 5, **dadurch gekennzeichnet, dass** vor und/oder nach der Blutegelextraktbestrahlung ein transparentes Medium durch die Anlage gefördert und die Intensität der in das Medium eingetragenen oder durch das Medium durchgetretenen Strahlung gemessen wird.

7. Verfahren nach einem der Ansprüche 1 bis 6, **dadurch gekennzeichnet, dass** das Volumenverhältnis zwischen dem Rührkessel (15) und der Bestrahlungsvorrichtung (20/21) im Bereich von 1 bis 1000, bevorzugt im Bereich von 5 bis 500, besonders bevorzugt im Bereich von 10 bis 200 liegt.

8. Verfahren nach einem der Ansprüche 1 bis 7, **dadurch gekennzeichnet, dass** die Bestrahlungsvorrichtung durch eine oder mehrere vorzugsweise parallel geschaltete Wendelmodule (20/21) gebildet wird, wobei ein Wendelmodul eine Vorrichtung ist, die mindestens eine Quelle für ultraviolette Strahlung und mindestens einen helikal um eine Achse verlaufenden Kanal bereitstellt.

9. Verfahren nach Anspruch 8, **dadurch gekennzeichnet, dass** der helikal verlaufende Kanal (95) so angeordnet ist, dass er um eine Quelle für ultraviolette Strahlung (80) herumläuft.

10. Verfahren nach Anspruch 8 oder 9, **dadurch gekennzeichnet, dass** der Kanal (90) ein Querschnittsprofil aufweist, das auf mindestens einer Seite abgeflacht ist, und von dieser abgeflachten Seite bevorzugt ultraviolette Strahlung (80) in den Kanal eingebracht wird.

11. Verfahren nach einem der Ansprüche 8 bis 10, **dadurch gekennzeichnet, dass** das Querschnittsprofil des Kanals (90) D-förmig, rautenförmig oder rechteckig ist.

12. Verfahren nach einem der Ansprüche 8 bis 11, **dadurch gekennzeichnet, dass** das Wendelmodul (90) einen Hohlzylinder (100) umfasst, auf den ein Wendelschlauch kraft- oder formschlüssig aufgebracht ist (90/95) und in den eine Quelle für ultraviolette Strahlung eingebracht ist.

13. Verfahren nach einem der Ansprüche 8 bis 12, **dadurch gekennzeichnet, dass** das Wendelmodul so gestaltet ist, dass mindestens die bestrahlten Bauteile (90/95/100) als Einwegteile ausgeführt sind.

**Claims**

1. Method for inactivating viruses and/or bacteria in a medicinal-leech extract, **characterized in that** the medicinal-leech extract is circulated between a stirred vessel and an irradiation device in which the medium is irradiated using ultraviolet light.

**2.** Method according to Claim 1, **characterized in that** irradiation is carried out using ultraviolet light in the range of 100 nm to 280 nm, preferably in the range of 200 nm to 280 nm.

**3.** Method according to Claim 1 or 2, **characterized in that** the medicinal-leech extract has an optical density in the range of up to 72, preferably in the range of 30 to 65, particularly preferably in the range of 40 to 60, at a wavelength of 254 nm.

**4.** Method according to any of Claims 1 to 3, **characterized in that** the ratio of pump-circulated volumetric flow rate to total volume is in the range of 0.5 to 80 1/h, preferably in the range of 1 to 60 1/h, particularly preferably in the range of 3 to 45 1/h.

**5.** Method according to any of Claims 1 to 4, **characterized in that** the temperature of the medicinal-leech extract is maintained in the range of 2°C to 25°C, preferably in the range of 4°C to 20°C, particularly preferably in the range of 8°C to 15°C.

**6.** Method according to any of Claims 1 to 5, **characterized in that**, before and/or after medicinal-leech extract irradiation, a transparent medium is conveyed through the system and the intensity of the radiation introduced into the medium or passed through the medium is measured.

**7.** Method according to any of Claims 1 to 6, **characterized in that** the volume ratio between the stirred vessel (15) and the irradiation device (20/21) is in the range of 1 to 1000, preferably in the range of 5 to 500, particularly preferably in the range of 10 to 200.

**8.** Method according to any of Claims 1 to 7, **characterized in that** the irradiation device is formed by one or more preferably parallel-connected spiral modules (20/21), wherein a spiral module is a device providing at least one source of ultraviolet radiation and at least one channel winding helically around an axis.

**9.** Method according to Claim 8, **characterized in that** the helically wound channel (95) is arranged in such a way that it passes around a source of ultraviolet radiation (80).

**10.** Method according to Claim 8 or 9, **characterized in that** the channel (90) has a cross-sectional profile which is flattened on at least one side, and ultraviolet radiation (80) is preferably introduced into the channel from this flattened side.

**11.** Method according to any of Claims 8 to 10, **characterized in that** the cross-sectional profile of the channel (90) is D-shaped, rhombus-shaped or rectangular.

**12.** Method according to any of Claims 8 to 11, **characterized in that** the spiral module (90) comprises a hollow cylinder (100) onto which spiral tubing is mounted in a force-fitted or form-fitted manner (90/95) and into which a source of ultraviolet radiation is introduced.

**13.** Method according to any of Claims 8 to 12, **characterized in that** the spiral module is designed such that at least the irradiated components (90/95/100) are used as disposable parts.

## Revendications

**1.** Procédé d'inactivation de virus et/ou de bactéries dans un extrait gélifié de sang,
**caractérisé en ce que**
l'extrait gélifié de sang est mis en circulation entre une cuve brassée et un dispositif d'irradiation dans lequel le milieu est exposé à une irradiation par de la lumière ultraviolette.

**2.** Procédé selon la revendication 1, **caractérisé en ce que** l'irradiation est réalisée avec de la lumière dans la plage de 100 nm à 280 nm et de préférence dans la plage de 200 nm à 280 nm.

**3.** Procédé selon l'une des revendications 1 ou 2, **caractérisé en ce que** l'extrait gélifié de sang présente à une longueur d'onde de 254 nm une densité optique pouvant atteindre 72, de préférence de l'ordre de 30 à 65 et de façon particulièrement préférable de l'ordre de 40 à 60.

**4.** Procédé selon l'une des revendications 1 à 3, **caractérisé en ce que** le rapport entre le débit volumique pompé et le volume total est de l'ordre de 0,5 à 80 1/h, de préférence de l'ordre de 1 à 60 1/h et de façon particulièrement préférable de l'ordre de 3 à 45 1/h.

**5.** Procédé selon l'une des revendications 1 à 4, **caractérisé en ce que** la température de l'extrait gélifié de sang est maintenue dans la plage de 2°C à 25°C, de préférence dans la plage de 4°C à 20°C et de façon particulièrement préférable dans la plage de 8°C à 15°C.

**6.** Procédé selon l'une des revendications 1 à 5, **caractérisé en ce qu'**avant et/ou après l'irradiation de l'extrait gélifié de sang, un fluide transparent est transporté dans l'installation et l'intensité du rayonnement introduit dans le fluide ou qui traverse le fluide est mesurée.

**7.** Procédé selon l'une des revendications 1 à 6, **caractérisé en ce que** le rapport volumique entre la cuve brassée (15) et le dispositif d'irradiation (20/21) est de l'ordre de 1 à 1 000, de préférence de l'ordre de 5 à 500 et de façon particulièrement préférable de l'ordre de 10 à 200.

**8.** Procédé selon l'une des revendications 1 à 7, **caractérisé en ce que** le dispositif d'irradiation est formé d'un ou de plusieurs modules en spirale (20/21) raccordés de préférence en parallèle, un module en spirale étant un dispositif qui présente une source de rayonnement ultraviolet et au moins un canal qui s'étend en hélice autour d'un axe.

**9.** Procédé selon la revendication 8, **caractérisé en ce que** le canal (95) qui s'étend en hélice est disposé de manière à entourer une source de rayonnement ultraviolet (80).

**10.** Procédé selon l'une des revendications 8 ou 9, **caractérisé en ce que** le canal (90) présente une section transversale dont le profil est aplati au moins sur un côté, le rayonnement ultraviolet (80) étant apporté dans le canal de préférence par ce côté aplati.

**11.** Procédé selon l'une des revendications 8 à 10, **caractérisé en ce que** le profil de la section transversale du canal (90) est en forme de D, de losange ou de rectangle.

**12.** Procédé selon l'une des revendications 8 à 11, **caractérisé en ce que** le module (90) en spirale comporte un cylindre creux (100) sur lequel un tube flexible spiralé et appliqué en correspondance mécanique ou en correspondance géométrique (90/95) et dans lequel une source de rayonnement ultraviolet est placée.

**13.** Procédé selon l'une des revendications 8 à 12, **caractérisé en ce que** le module en spirale est configuré avec au moins les composants irradiés (90/95/100) réalisés comme pièces à usage unique.

**Fig. 1**

## Blutegelextraktionsprozess

```
┌─────────────────────────────────────┐
│          gefrorene Blutegel          │
└─────────────────────────────────────┘
                   │
                   ▼
┌─────────────────────────────────────┐
│          Zermalen / Auftauen         │
└─────────────────────────────────────┘
                   │
                   ▼
┌─────────────────────────────────────┐
│     Erste Aceton-Salz Extraktion     │──── Überstand
│            Zentrifugation            │
└─────────────────────────────────────┘
   Präzipitat      │
                   ▼
┌─────────────────────────────────────┐
│     Zweite Aceton-Salz Extraktion    │──── Überstand
│            Zentrifugation            │
└─────────────────────────────────────┘
   Präzipitat      │
                   ▼
┌─────────────────────────────────────┐
│     Dritte Aceton-Salz Extraktion    │──── Überstand
│            Zentrifugation            │
└─────────────────────────────────────┘

Verwerfen des ◄──────
Präzipitats
                   │
                   ▼
┌─────────────────────────────────────┐
│              Filtration              │
│     Einstellen des pH-Werts auf 4,5  │
└─────────────────────────────────────┘
                   │
                   ▼
┌─────────────────────────────────────┐
│            Aceton-Fällung            │
└─────────────────────────────────────┘
                   │
                   ▼
┌─────────────────────────────────────┐
│     Absetzen von Protein-Präzipitat  │
└─────────────────────────────────────┘
                   │
                   ▼
┌─────────────────────────────────────┐
│        Waschen des Präzipitats       │
└─────────────────────────────────────┘
                   │
                   ▼
┌─────────────────────────────────────┐
│  Isolierung des Präzipitats durch Filtration │
└─────────────────────────────────────┘
                   │
                   ▼
┌─────────────────────────────────────┐
│   Vakuumtrockung des Filterkuchens   │
└─────────────────────────────────────┘
                   │
                   ▼
┌─────────────────────────────────────┐
│          Roher Blutegelextrakt       │
└─────────────────────────────────────┘
```

**Fig. 2**

## Herstellung von Blutegelextrakt (gefriergetrocknet)

| zusammengeführte Rohextrakte |
|:---:|

| Auflösen in Wasser |
|:---:|

| Einfrieren / Lagerung der Lösung |
|:---:|

| Auftauen / Mischen mit Wasser |
|:---:|

| Einstellen eines pH-Werts von 7,5 |
|:---:|

| Zentrifugation | → Überstand |
|:---:|:---|

Präzipitat

| Waschen des Präzipitats | → Überstand |
|:---:|:---|

Verwerfen des Präzipitats ←

| Filtration |
|:---:|

| UVC-Bestrahlung |
|:---:|

| Filtration |
|:---:|

| Konzentrierung durch Ultrafiltration |
|:---:|

| Filtration |
|:---:|

| Abfüllen und Gefriertrocknen |
|:---:|

| Gefriergetrockneter Blutegelextrakt |
|:---:|

**Fig. 3**

**Fig. 4**

**Fig. 5**

**Fig. 6**

**Fig. 7**

Legend:
- ● Virus-Inaktivierung
- △ Hirudin- Inaktiverung

X-Achse: Strahlendosis [J/m$^2$]
Linke Y-Achse: Virus-Inaktivierung [log N/N$_0$]
Rechte Y-Achse: Hirudin-Inaktivierung [%]

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- EP 1339643 A1
- EP 1337280 A1
- WO 2002038502 A1
- WO 0238502 A1
- WO 0238191 A1

- WO 07096057 A1
- EP 1464342 A1
- DE 102009009108
- WO 07096057 A2

**In der Beschreibung aufgeführte Nicht-Patentliteratur**

- **NOWAK, G. ; SCHRÖR, K.** Hirudin - the long and stony way from an anticoagulant peptide in the saliva of medicinal leech to a recombinant drug and beyond. A historical piece. *Thromb. Haemost.,* 2007, vol. 98, 116-119